# EUROPÄISCHE PATENTSCHRIFT

(11) **EP 3 439 568 B1**
(45) Veröffentlichungstag und Bekanntmachung des Hinweises auf die Patenterteilung: **02.03.2022**
(21) Anmeldenummer: 17716831.7
(22) Anmeldetag: 06.04.2017
(51) Int. Cl.: A61B 18/12, A61B 18/14, A61B 17/29

(54) **SCHAFT FÜR EIN MEDIZINISCHES INSTRUMENT SOWIE MEDIZINISCHES INSTRUMENT, INSBESONDERE MONO- ODER BIPOLARES MEDIZINISCHES INSTRUMENT**
SHAFT FOR A MEDICAL INSTRUMENT, AND MEDICAL INSTRUMENT, IN PARTICULAR HOMOPOLAR OR BIPOLAR MEDICAL INSTRUMENT
TIGE POUR INSTRUMENT MÉDICAL AINSI QU'INSTRUMENT MÉDICAL, EN PARTICULIER INSTRUMENT MÉDICAL MONOPOLAIRE OU BIPOLAIRE

(30) Priorität: 07.04.2016 DE 102016106397
(43) Veröffentlichungstag der Anmeldung: 13.02.2019
(60) Teilanmeldung: 19184482.8
(73) Patentinhaber: Tontarra Medizintechnik GmbH, 78573 Wurmlingen (DE)
(72) Erfinder: BENK, Michael, 78573 Wurmlingen (DE)
(74) Vertreter: Patentanwälte Mammel und Maser PartG mbB
(86) Internationale Anmeldenummer: PCT/EP2017/058217
(87) Internationale Veröffentlichungsnummer: WO 2017/174707

(56) Entgegenhaltungen:
- EP-A1- 2 653 117
- DE-A1-102006 056 405
- US-A1- 2015 105 820

## Beschreibung

Schaft für ein medizinisches Instrument sowie medizinisches Instrument, insbesondere mono- oder bipolares medizinisches Instrument

Die Erfindung betrifft einen Schaft für ein medizinisches Instrument sowie ein medizinisches Instrument, insbesondere mono- oder bipolares medizinisches Instrument.

Aus der DE 101 56 917 A1 ist ein medizinisches Instrument für die endoskopische Chirurgie bekannt. Ein rohrförmiger Schaft ist an seinem proximalen Ende an einem Gehäuse befestigt, welches Griffteile aufweist, um eine in dem rohrförmigen Schaft geführte Zug-/Druckstange zu betätigen. Durch diese wird ein am distalen Ende des Schaftes angeordnetes Werkzeug mit zwei Maulteilen aus einer Öffnungsstellung in eine Schließstellung oder Greifstellung übergeführt. Die beiden das Werkzeug bildenden Maulteile sind durch eine gemeinsame Schwenkachse im Schaft schwenkbar gelagert, welche ortsfest an dem Schaft angeordnet ist. Während dem Öffnen und Schließen der Maulteile ragen Schwenkarme der Maulteile beziehungsweise sogenannte Angeln gegenüber einem Außenumfang des Schaftkörpers heraus. Eine solche Anordnung weist den Nachteil auf, dass die jeweils mit Strom beaufschlagten Maulteile und die gegenüber dem Schaftkörper herausragenden Angeln zu Verletzungen nahe dem Arbeitsbereich der Maulteile führen können.

Aus der DE 20 2004 010 780 U1 ist ein analoges chirurgisches Instrument für die Elektrochirurgie bekannt.

Analoges gilt auch für ein elektrisch chirurgisches Instrument gemäß der DE 20 2011 052 418 U1, bei welchem nur ein Gelenkarm des bewegbaren Maulteils beim Überführen in eine Öffnungsbewegung gegenüber einem zylindrischen Schaftkörper nach außen hervortritt. Das Dokument DE 10 2006 056405 A1 offenbart die Grundlage für den Oberbegriff von Anspruch 1.

Der Erfindung liegt die Aufgabe zugrunde, einen Schaft für ein medizinisches Instrument sowie ein medizinisches Instrument, insbesondere ein mono- oder bipolares medizinisches Instrument, für die Elektrochirurgie zu schaffen, bei welchem während der Öffnungs- und Schließbewegung des zumindest einen Maulteils Betätigungsglieder zum Einleiten der Öffnungs- und Schließbewegung der Maulteile innerhalb eines Schaftes verbleiben.

Diese Aufgabe wird durch einen Schaft für ein medizinisches Instrument gelöst, bei welchem das zumindest eine Maulteil am distalen Ende im Schaftkörper durch zumindest eine Kulisse schwenkbar geführt ist, wobei eine bogensegmentförmige Laufbahn und eine koaxial dazu verlaufende Führungsbahn vorgesehen ist und ein Betätigungsglied des Maulteils komplementär gekrümmt ausgebildet ist und mit einer äußeren und inneren Führungsfläche entlang der Laufbahn und der Führungsbahn geführt ist, wobei im Schaftkörper ein Schlitten entlang der Längsachse des Schaftkörpers verfahrbar geführt ist, der durch die Zug-/Druckstange bewegbar ist und das zumindest eine Maulteil in eine Öffnungsstellung und eine Schließstellung überführt. Durch eine solche Kulisse kann eine bahn- oder kurvengesteuerte Öffnungs- und Schließbewegung des oder der Maulteile erzielt werden, ohne dass Bestandteile des oder der Maulteile, wie beispielsweise Betätigungsglieder, welche für die Öffnungs- und Schließbewegung angesteuert werden, gegenüber einem Außenumfang des Schaftes hervorstehen. Durch die kulissengeführte oder bahngesteuerte Öffnungs- und Schließbewegung des zumindest einen Maulteils kann ein geschlossener Schaftkörper vom proximalen Ende bis zum distalen Ende vorgesehen sein, so dass lediglich die Arbeitsbereiche der Maulteile distal außerhalb des Schaftkörpers vorgesehen sind. Jegliche Antriebskomponenten für die Durchführung der Öffnungs- und Schließbewegung des zumindest einen Maulteils sind innerhalb eines geschlossenen Schaftkörpers vorgesehen. Diese gelenkachsenfreie Aufnahme des oder der Maulteile ermöglicht die in Umfangsrichtung nach außen geschlossene Ausbildung des Schaftkörpers. Insbesondere beim Einsatz für ein mono- oder bipolares medizinisches Instrument wird dadurch sichergestellt, dass ein Stromfluss ausschließlich im Arbeitsbereich der Maulteile erfolgt, da keine stromführenden Bestandteile der Maulteile oder eines Antriebs der Maulteile seitlich beziehungsweise radial aus der Außenumfangsfläche des Schaftes heraustreten.

Des Weiteren ist bevorzugt vorgesehen, dass für jedes Maulteil am distalen Ende im Schaftkörper jeweils eine Kulisse zur getrennten Aufnahme vorgesehen ist. Dies ermöglicht, dass jedes an dem Maulteil vorgesehene Betätigungsglied getrennt in einer Kulisse geführt werden kann. Dabei kann vorgesehen sein, dass die Kulissen unmittelbar aneinander grenzen und teilweise einen gemeinsamen Kulissenraum bilden. Dadurch kann eine besonders platzsparende Anordnung geschaffen werden, um insbesondere dünne Durchmesser für die minimalinvasive Chirurgie zu schaffen. In diesem Fall können die gegebenenfalls aneinander liegenden Abschnitte der Betätigungsglieder mit einer Isolierung oder einer isolierenden Beschichtung versehen sein. Vorzugsweise ist vorgesehen, dass jede Kulisse innerhalb eines Außenumfangs des Schaftkörpers angeordnet ist. Dadurch wird verhindert, dass das Betätigungsglied des Maulteils gegenüber einem Außenumfang des Schaftkörpers heraustritt und den Durchmesser der Schaftkörper über die gesamte Länge beibehalten werden können.

Bevorzugt ist vorgesehen, dass zwei zueinander benachbarte und getrennt zueinander angeordnete Kulissen am distalen Ende des Schaftkörpers vorgesehen sind, in welcher jeweils ein Betätigungsglied des Maulteils angeordnet ist. Dies weist den Vorteil auf, dass die Betätigungsglieder, welche innerhalb des Schaftkörpers in der jeweiligen Kulisse positioniert und verschiebbar geführt sind, getrennt zueinander angeordnet sind. Bei einem mono- oder bipolaren medizinischen Instrument kann eine erleichterte Stromführung für jedes Maulteil ermöglicht sein.

Bevorzugt weist die zumindest eine Kulisse eine Austrittsöffnung auf, welche in einer Stirnfläche des Schaftkörpers liegt. Dadurch kann die benachbarte Außenumfangsfläche des Schaftkörpers geschlossen sein. Zudem ist die Stirnfläche des Schaftkörpers mit Ausnahme der zumindest einen zur zumindest einen Kulisse führenden Öffnung geschlossen. Das Betätigungsglied des Maulteils wird durch diese Öffnung hindurch in der Kulisse geführt.

Eine weitere bevorzugte Ausgestaltung des Schaftes sieht vor, dass die Kulisse eine bogensegmentförmige Laufbahn und eine koaxial dazu verlaufende Führungsbahn aufweist und das Betätigungsglied komplementär gekrümmt ausgebildet ist und mit einer inneren und äußeren Führungsfläche entlang der Laufbahn und Führungsbahn geführt ist. Durch diese bogensegmentförmig gebildete Laufbahn kann bei einer auf das Betätigungsglied wirkenden Verschiebebewegung in und entgegen der Längsachse des Schaftes ein Öffnen und Schließen der Maulteile erzielt werden. Beim Öffnen der Maulteile wird lediglich ein Teilbereich des Betätigungsgliedes vom Maulteil aus dem distalen Ende des Schaftkörpers herausbewegt oder herausgeführt, jedoch im Übrigen kann eine Stirnfläche des Schaftkörpers am distalen Ende geschlossen ausgebildet sein.

Des Weiteren ist bevorzugt vorgesehen, dass die beiden einander gegenüberliegenden Kulissen punktsymmetrisch zur Längsachse des Schaftkörpers ausgerichtet sind. Dadurch wird ermöglicht, dass ein synchroner Verlauf beim Öffnen und Schließen der beiden Maulteile bei den zueinander angeordneten Kulissen ermöglicht ist.

Alternativ kann vorgesehen sein, dass die einander gegenüberliegenden Kulissen voneinander abweichende Verläufe aufweisen. Dadurch kann eine asynchrone Bewegung der Maulteile erzielt werden. Beispielsweise kann ein Maulteil einen größeren Bewegungsumfang als das gegenüberliegende Maulteil aufweisen. Darüber hinaus kann ein Maulteil zu einem anderen Zeitpunkt als das zumindest eine weitere Maulteil in eine Schiefstellung übergeführt werden.

Des Weiteren ist bevorzugt vorgesehen, dass die Betätigungsglieder der beiden Maulteile gegenläufig gekrümmt sind. In Abhängigkeit der Ausgestaltung des Arbeitsbereiches der Maulteile können dadurch Gleichteile gegeben sein, sofern beispielsweise ein schneidender oder ein klemmender Arbeitsbereich vorgesehen ist, können für die Maulteile Gleichteile vorgesehen sein. Sofern bei einem greifenden Arbeitsbereich eine Verzahnung oder Verrippung vorgesehen ist, ist der grundsätzliche Aufbau der Maulteile mit Ausnahme der Verzahnung und Verrippung wiederum identisch.

Die Kulisse ist vorteilhafterweise innerhalb dem Schaftkörper geschlossen ausgebildet. Diese kann einen rechteckigen, quadratischen, runden, ovalen oder halbkreisförmigen Querschnitt aufweisen. Dadurch wird das Maulteil verdrehsicher geführt. Eine Öffnungs- und Schließbewegung kann dadurch gezielt angesteuert werden. Gleichzeitig wird bei einem Schließen der Maulteile und einem Greifen von einem Gewebe ein Verdrehen oder Verkippen der Maulteile zueinander verhindert.

Des Weiteren ist bevorzugt vorgesehen, dass das Betätigungsglied des Maulteils stabförmig und gekrümmt ausgebildet ist. Dieses weist bevorzugt einen komplementären Verlauf und Geometrie zur Kulisse auf. Dadurch kann in einfacher Weise eine Führung in der Kulisse erfolgen. Dadurch kann eine sehr einfache Führung gegeben sein.

Das Betätigungsglied des Maulteils ist bevorzugt außermittig zur Längsachse des Arbeitsbereiches des Maulteils vorgesehen. Dies ist bevorzugt dann gegeben, wenn die Arbeitsbereiche breit sind wie beispielsweise bei einer greifenden oder klemmenden Funktion. Des Weiteren kann vorgesehen sein, dass das Betätigungsglied des Maulteils seitlich versetzt zum Arbeitsbereich des Maulteils vorgesehen ist. Insbesondere bei einer schneidenden Funktion ist dies gegeben, so dass wieder eine einfache Anbindung der beiden Scherenteile an dem Schaft ermöglicht ist.

An dem Ende des Betätigungsgliedes der Maulteile ist vorzugsweise ein Führungszapfen vorgesehen, der in eine Führungsnut an einem Schlitten eingreift, welcher durch die Zug-/Druckstange im Schaftkörper bewegbar ist. Dies ermöglicht eine einfache Kopplung eines Schlittens mit dem zumindest einen Führungszapfen von dem zumindest einen bewegbaren Maulteil vorzugsweise von den beiden Führungszapfen des jeweiligen Betätigungsgliedes, so dass in einfacher Weise durch eine Schiebebewegung des Schlittens ein Öffnen und Schließen des oder der Maulteile gegeben ist. Zudem ermöglicht dies eine einfache Montage.

Der Schlitten innerhalb des Schaftkörpers weist bevorzugt zwei einander gegenüberliegende und nach außen offene Führungsnuten auf und der Schlitten ist im Bereich der Führungsnuten zwischen den beiden Kulissen verfahrbar geführt. Dadurch ist ein bauraumsparender Antrieb für die Maulteile gegeben. Dies ermöglicht des Weiteren das gleichzeitige Ansteuern der Betätigungsglieder der Maulteile als auch eine einfache konstruktive Ausgestaltung.

Des Weiteren ist bevorzugt vorgesehen, dass die Führungsnuten des Schlittens rechtwinklig zur Arbeitsbewegung oder Verfahrbewegung des Schlittens entlang der Längsachse im Schaftkörper ausgerichtet sind. Dadurch können Schub- und Zugkräfte in einfacher Weise übertragen werden, um das Öffnen und Schließen des Maulteils oder der Maulteile anzusteuern.

Der Schlitten im Schaftkörper ist bevorzugt als ein länglicher Führungskörper ausgebildet, der verdrehsicher in dem Schaftkörper verfahrbar geführt ist. Vorteilhafterweise ist ein quadratischer Querschnitt, insbesondere mit gerundeten Kanten vorgesehen, so dass dieser im Schaftkörper nicht verkantet und dennoch verdrehsicher angeordnet ist.

Die Maulteile können aus Metall, Kunststoff, Keramik oder dergleichen bestehen. Sofern die Maulteile für ein monopolares oder bipolares Instrument vorgesehen sind, können die nichtleitenden Materialien der Maulteile zumindest bereichsweise mit einer leitenden Beschichtung oder leitenden Bestandteilen versehen sein. In Abhängigkeit des Anwendungsfalles und der Ausgestaltung, ob es sich um ein Einweg- oder Mehrweg-Schaft handelt, können diese Materialien für die Maulteile ausgewählt werden. Alternativ kann auch eine leitende Beschichtung aufgetragen werden.

Zur Bestromung des oder der Maulteile bei einem mono- oder bipolaren medizinischen Instrument ist bevorzugt vorgesehen, dass zumindest eine gekrümmte Außenfläche des Betätigungsgliedes eine Kontaktstelle für eine Elektrode bildet. Dadurch kann unabhängig der Öffnungs- und Schließbewegung eine Kontaktierung gegeben sein. Alternativ kann vorgesehen sein, dass beispielsweise nur ein Endbereich des Betätigungsgliedes mit einer leitenden Beschichtung ausgebildet ist oder mit einem leitenden Bereich ausgebildet ist, der mit der Greiffläche des Maulteils leitend verbunden ist. Dadurch wird ermöglicht, dass beispielsweise erst bei einer Endphase der Schließbewegung oder nach dem die Maulteile nahezu geschlossen sind eine Bestromung der Maulteile erfolgt. Somit können die geöffneten Maulteile unbestromt bleiben.

Bevorzugt ist vorgesehen, dass die im Schaftkörper vorgesehene zumindest eine Elektrode getrennt zum verfahrbaren Schlitten angeordnet ist. Dadurch kann eine Öffnungs- und Schließbewegung der Maulteile erfolgen, ohne dass dabei gleichzeitig auch eine Betätigung oder Bewegung der zumindest einen Elektrode erforderlich ist. Dadurch wird die Anzahl der bewegten Teile im Schaftkörper reduziert.

Bevorzugt ist vorgesehen, dass in dem Schaftkörper ein oder zwei elektrische Leitungen mit jeweils einem Kontaktelement, insbesondere einem Schleifkontakt vorgesehen sind, der mit dem Betätigungsglied des Maulteils kontaktiert ist. Solche Schleifkontakte übertragen während einer Öffnungs- und Schließbewegung des Maulteils den Strom. Gleichzeitig sind solche Schleifkontakte verschleißarm. Bevorzugt ist der Schleifkontakt als ein gebogenes metallisches Federelement ausgebildet, so dass dieser Schleifkontakt unter Vorspannung an dem Betätigungsglied des Maulteils anliegt.

Des Weiteren ist bevorzugt vorgesehen, dass die Maulteile einen schneidenden, greifenden, klemmenden, löffelmaulförmigen Arbeitsbereich aufweisen oder als Pinzette oder Nadelelektrode ausgebildet sind.

Ausführungsbeispiele des Instruments umfassen einen Schaft, der einen Schaftkörper aus einer ersten und einer zweiten Körperhälfte umfasst, welche halbschalenförmig ausgebildet sind und die zu einem geschlossenen Schaftkörper verbindbar sind. Diese zweiteilige Ausgestaltung des Schaftkörpers ermöglicht, dass eine einfache Montage der Komponenten gegeben ist, in dem beispielsweise eine Zug-/Druckstange und ein damit gekoppelter Schlitten sowie ein oder zwei bewegliche Maulteile, welche mittels dem Schlitten zum Öffnen und Schließen angetrieben werden, in eine erste Körperhälfte eingelegt werden, um darauffolgend die zweite Körperhälfte mit der ersten Körperhälfte zu verbinden und einen vollständig geschlossenen Schaftkörper vom proximalen bis zum distalen Ende, insbesondere bezüglich einer äußeren Umfangsfläche des Schaftes zu erzielen. Sofern der Schaft für ein mono- oder bipolares medizinisches Instrument eingesetzt wird, können vor dem Zusammenführen der beiden Körperhälften eine oder beide Elektroden eingelegt beziehungsweise eingesetzt werden.

Des Weiteren ist bevorzugt vorgesehen, dass die erste und zweite Körperhälfte im Wesentlichen entlang der Längsmittelebene des Schaftes getrennt sind. Dies ermöglicht, dass sowohl mit der ein oder der anderen Körperhälfte bei der Montage begonnen werden kann, ohne dass eine Bevorzugung von einer der beiden Körperhälften erforderlich wäre.

Bevorzugt ist die erste und zweite Körperhälfte des Schaftkörpers identisch ausgebildet. Dies ermöglicht eine Kostenreduzierung bei der Herstellung.

Vorteilhafterweise können die beiden Körperhälften durch eine Rast- oder Klipsverbindung zueinander fixierbar sein. Dies ermöglicht eine einfache Verbindung der beiden Körperhälften und schafft des Weiteren eine absatzfreie und geschlossene Außenumfangsfläche, insbesondere auch entlang den Längskanten der aneinander liegenden Körperhälften.

Eine weitere bevorzugte Ausgestaltung des Schaftes sieht vor, dass die erste und zweite Körperhälfte als Spritzgussteil, insbesondere aus einem Kunststoff hergestellt ist. Bevorzugt kann ein thermoplastischer Kunststoff, insbesondere Polyoxymethylen (POM) oder dergleichen eingesetzt werden. Dadurch kann eine kostengünstige Herstellung eines Einwegschaftes gegeben sein. Alternativ kann vorgesehen sein, dass die erste und zweite Körperhälfte auch aus einer Keramik ausgebildet ist.

Die erste und zweite Körperhälfte weisen jeweils eine zentrale Ausnehmung entlang der Längsmittelachse zur Aufnahme der Zug-/Druckstange auf. Dies stellt die einfachste Ausführungsform dar, sofern innerhalb des Schaftes nur die Zug-/Druckstange aufzunehmen ist.

Für ein mono- oder bipolares Instrument kann die erste und zweite Körperhälfte des Schaftes benachbart zur zentralen Ausnehmung eine Vertiefung zur Aufnahme einer Elektrode aufweisen und gegenüber liegend auf der anderen Seite der Aufnahme eine Erhöhung umfassen. Dadurch werden der oder die Elektroden beim Zusammenführen der beiden Körperhälften in der jeweiligen Vertiefung automatisch fixiert, da die Erhöhung der einen Körperhälfte in die Vertiefung der anderen Körperhälfte zumindest teilweise eingreift.

Des Weiteren ist bevorzugt entlang einer ersten Längsseite ein positives Rastelement und entlang einer zweiten Längsseite ein negatives Rastelement vorgesehen. Dadurch kann eine einfache Steck-, Rastbeziehungsweise Clipsverbindung zur Verbindung der beiden Körperhälften zu einem Schaft erfolgen. Zudem können solche Verbindungen in einfacher Weise auch aus Kunststoff, insbesondere als Spritzgussteil, ausgebildet werden. Durch diesen vorbeschriebenen Aufbau kann die erste und zweite Körperhälfte identisch ausgebildet und dennoch zu einem gemeinsamen Schaftkörper zusammengefügt werden, wobei das positive und negative Rastelement eine Rastverbindung bilden. Benachbart zur zentralen Ausnehmung der Zug-/Druckstange ist nach dem Zusammensetzen des Schaftkörpers jeweils getrennt eine geschlossene Aufnahme für die Elektrode gebildet, die vom proximalen Ende bis zum distalen Ende geführt wird und am distalen Ende mit dem jeweiligen Maulteil kontaktiert ist.

Des Weiteren ist bevorzugt am proximalen Endbereich der ersten und zweiten Körperhälfte jeweils eine Hälfte des Anschlussstücks angeformt, welche durch weitere Rastelemente miteinander verbindbar sind. Dadurch kann der gesamte Schaft aus zwei Körperhälften ausgebildet werden und dennoch ohne zusätzliche Bauteile eine Aufnahme an der Handhabungseinrichtung ermöglichen. Über dieses Anschlussstück kann der Schaft durch eine Clips-, Rast- oder Steckverbindung mit der Handhabungseinrichtung verbunden sein. Gleichzeitig kann der Schaft um seine Längsachse drehbar zur Handhabungseinrichtung aufgenommen werden.

Eine weitere bevorzugte Ausgestaltung des Schaftes sieht vor, dass das proximale Ende des Schaftes einen Führungsabschnitt aufweist, durch welchen der Schaft geführt in der Handhabungseinrichtung, insbesondere drehbar, aufgenommen ist.

Des Weiteren ist bevorzugt an jeder Hälfte des Anschlussstückes und/oder des Führungsabschnitts zumindest eine Aussparung vorgesehen, in welcher ein Kontaktelement, insbesondere ein klammerförmiges, laschenförmiges oder stiftförmiges, Kontaktelement einsetzbar ist, das mit der Elektrode leitend verbunden ist, wobei zumindest ein Kontaktelement an einer Außenumfangswand des Führungsabschnitts liegt. Dadurch kann in einfacher Weise nach dem Einsetzen des proximalen Endbereichs des Schaftes in die Handhabungseinrichtung nicht nur eine Fixierung, sondern gleichzeitig eine Kontaktierung mit einem elektrischen Anschlusskontakt auch unter Beibehaltung einer drehbaren Aufnahme des Schaftes zur Handhabungseinrichtung ermöglicht sein.

Des Weiteren ist bevorzugt der Zug-/Druckstab am proximalen Ende gegenüber dem Führungsabschnitt herausgeführt und weist vorzugsweise ein Anschlusselement auf, welches mit einem bewegbaren Handgriff der Handhabungseinrichtung verrastbar ist. Dadurch kann eine einfache Anbindung des Zug-/Druckstabs an den beweglichen Handgriff gegeben sein, um daraufhin die Maulteile zu betätigen.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
Figur 1 eine perspektivische Ansicht des erfindungsgemäßen Schaftes für ein mono- oder bipolares medizinisches Instrument,
Figur 2 eine schematische Ansicht eines bipolaren medizinischen Instruments,
Figur 3 eine schematische Ansicht eines distalen Ende des Schaftes gemäß Figur 1 mit einem Werkzeug in Schließstellung,
Figur 4 eine perspektivische Ansicht des Werkzeugs gemäß Figur 3 in einer Öffnungsstellung,
Figur 5 eine erste perspektivische Ansicht eines Maulteils,
Figur 6 eine zweite perspektivische Ansicht eines Maulteils,
Figur 7 eine vergrößerte Darstellung eines distalen Endes von einer ersten Körperhälfte des Schaftes,
Figur 8 eine perspektivische Ansicht eines größeren distalen Bereichs der ersten Körperhälfte des Schaftes,
Figur 9 eine perspektivische Ansicht eines distalen Ende des Schaftes gemäß Figur 3 mit einer abgenommenen oberen Körperhälfte des Schaftes,
Figur 10 eine perspektivische Ansicht gemäß Figur 9 mit dem Werkzeug in Öffnungsposition,
Figur 11 eine schematische Seitenansicht im Querschnitt des distalen Bereichs des Schaftes gemäß Figur 3,
Figur 12 eine perspektivische Ansicht einer ersten Körperhälfte des Schaftkörpers im mittleren Bereich des Schaftes,
Figur 13 eine schematische Schnittansicht entlang der Linie XIII-XIII in Figur 1,
Figur 14 eine schematische Ansicht eines proximalen Endbereichs einer ersten Körperhälfte des Schaftes,
Figur 15 eine schematische Teilansicht einer Druckstange mit Elektroden,
Figur 16 eine perspektivische Schnittansicht eines Anschlussbereiches vom Schaft und der Handhabungseinrichtung,
Figur 17 eine perspektivische Ansicht einer alternativen Ausführungsform eines Schaftes mit einem feststehenden und bewegbaren Maulteil in einer Schließposition,
Figur 18 eine perspektivische Ansicht der Ausführungsform gemäß Figur 17 mit einem geöffneten Maulteil,
Figur 19 eine schematische Ansicht von oben auf die Ausführungsform gemäß Figur 17 und
Figur 20 eine perspektivische Ansicht eines distalen Ende des Schaftes gemäß Figur 1 mit einem alternativen Werkzeug.

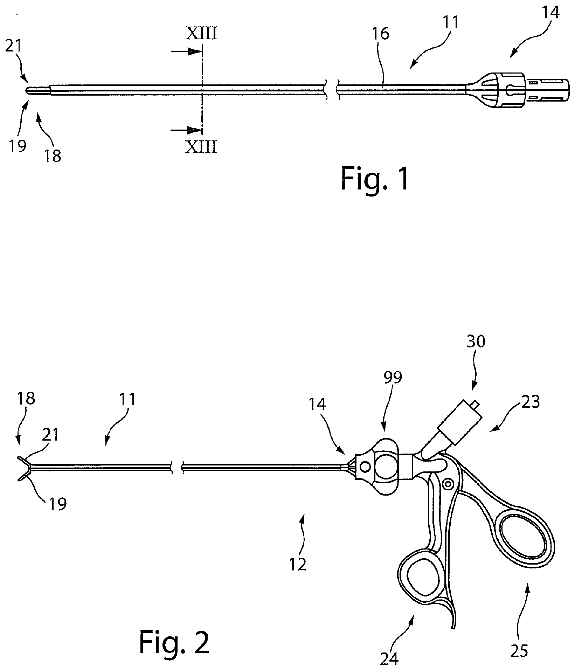

In Figur 1 ist ein Schaft 11 für ein chirurgisches Instrument 12 gemäß Figur 2 dargestellt. Dieser Schaft 11 umfasst am proximalen Ende ein Anschlussstück 14 sowie einen Schaftkörper 16, der sich zwischen dem Anschlussstück 14 und einem am distalen Ende des Schaftes 11 angeordneten Werkzeug 18 erstreckt. Das Werkzeug 18 umfasst ein erstes und zweites Maulteil 19, 21 die vorzugsweise beide zueinander bewegbar sind.

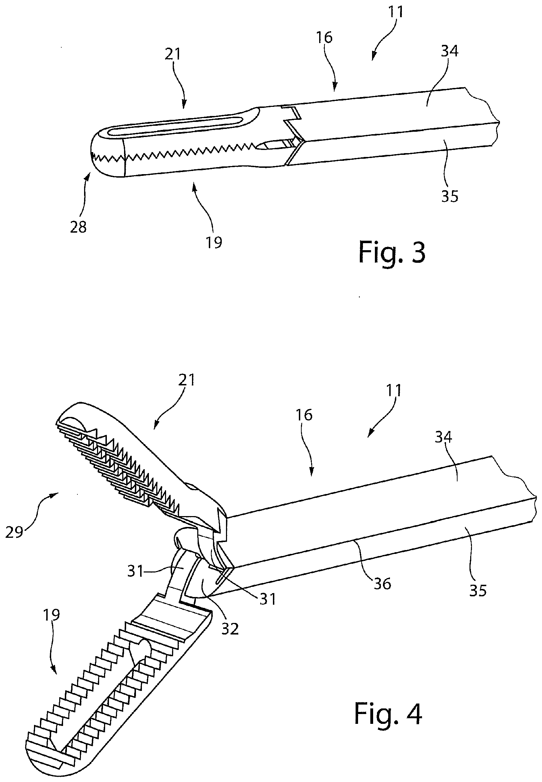

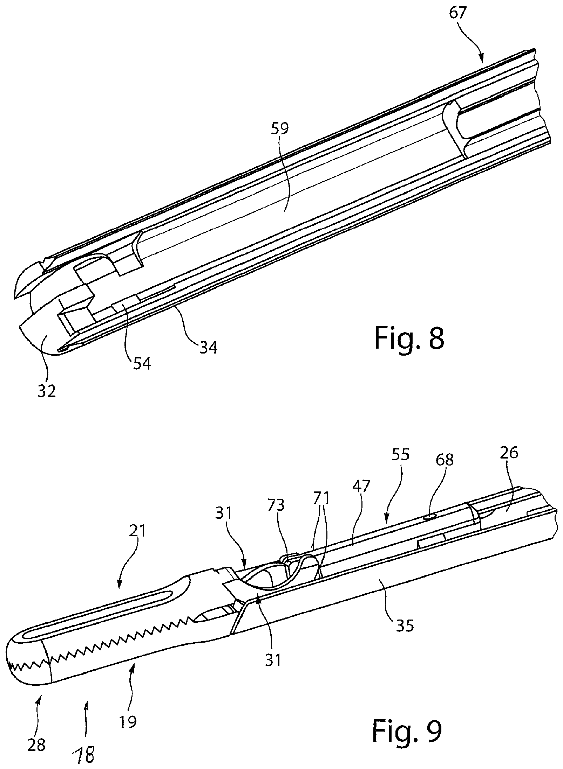

Ein solcher Schaft 11 ist für das chirurgische Instrument 12 gemäß Figur 2 einsetzbar und an einer Handhabungseinrichtung 23 befestigbar. Diese Handhabungseinrichtung 23 umfasst zwei Griffteile 24, 25 welche zueinander bewegbar sind, wodurch eine im Schaftkörper 16 angeordnete Zug-/Druckstange 26 (Figur 9) betätigbar ist, um das Werkzeug 18 aus einer Schließstellung 28 gemäß Figur 3 in eine Öffnungsstellung 29 gemäß Figur 4 überzuführen und anschließend wieder zu schließen.

Die Handhabungseinrichtung 23 kann einen Anschluss 30 aufweisen, an welchem ein Verbindungskabel einer nicht näher dargestellten Stromversorgung, beispielsweise einem Hochfrequenz-Chirurgiegerät, angeschlossen werden kann. Ein solcher Anschluss 30 kann als eine zweipolige Anschlussbuchse ausgebildet sein, das heißt, sie weist zwei gegeneinander elektrisch isolierte Pole für ein bipolares medizinisches Instrument 12 auf. Einer der Pole ist für das erste Maulteil 19 und der andere Pol für das zweite Maulteil 21 bestimmt.

Ebenso kann der Anschluss 30 auch nur eine einpolige Anschlussbuchse für ein monopolares medizinisches Instrument aufweisen.

Dieses chirurgische Instrument 12 kann im Rahmen der minimal-invasiven Chirurgie zur Behandlung von Gewebe im menschlichen oder tierischen Körper mit Hochfrequenzstrom eingesetzt werden. Das Instrument 12 ist sowohl monopolar als auch bipolar zu betreiben.

In Figur 3 ist schematisch vergrößert ein proximaler Endbereich des Schaftkörpers 16 mit dem ersten und zweiten Maulteil 19, 21 in einer Schließstellung 28 dargestellt. Der Schaftkörper 16 ist bis zu den Maulteilen 19, 21 geschlossen ausgebildet. Stirnseitig zum Schaftkörper 16 sind die Maulteile 19, 21 angebunden. Der Schaftkörper 16 weist eine geschlossene äußere Mantelfläche vom distalen bis zum proximalen Ende auf.

In Figur 4 ist perspektivisch ein distaler Bereich des Schaftkörpers 16 mit einem ersten und zweiten Maulteil 19, 21 in einer Öffnungsstellung 29 dargestellt. Das erste und zweite Maulteil 19, 21 weist jeweils ein Betätigungsglied 31 auf, welches gegenüber einer Stirnfläche 32 geringfügig herausgeführt sind, um die Maulteile 19, 21 in die Öffnungsposition überzuführen. Der Schaftkörper 16 ist zweiteilig ausgebildet und weist eine erste und zweite Körperhälfte 34, 35 auf. Diese weisen bevorzugt eine in der Längsmittelachse liegende Trennebene 36 auf, beziehungsweise die jeweiligen Längskanten der ersten und zweiten Körperhälfte 34, 35 liegen in der Trennebene 36.

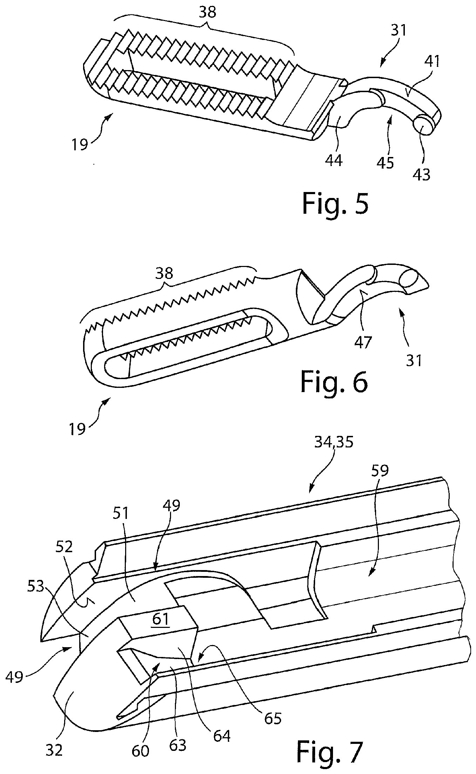

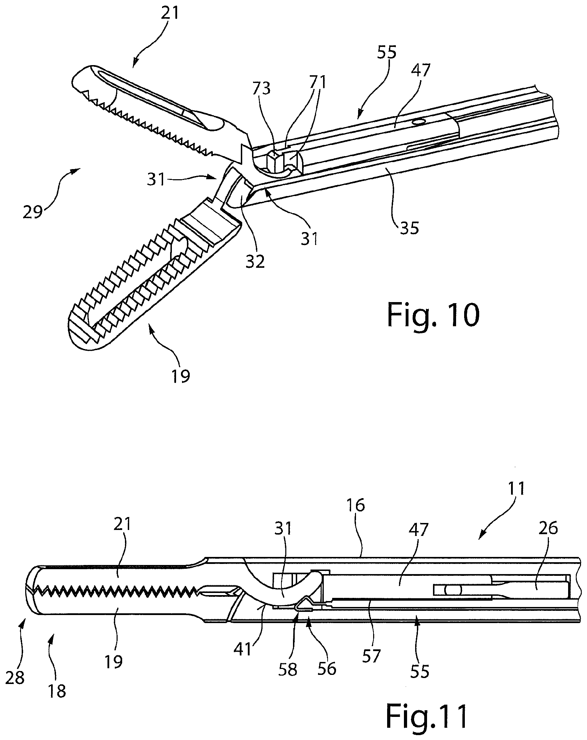

Das erste Maulteil 19 ist in einer perspektivischen Ansicht von oben gemäß Figur 5 in einer perspektivischen Ansicht von unten gemäß Figur 6 dargestellt. Dieses Maulteil 19 weist einen Arbeitsbereich 38 auf, der beispielsweise als Klemmbereich ausgebildet ist. Am Ende des Arbeitsbereiches schließt sich das Betätigungsglied 31 an, welches seitlich versetzt zur Längsmittelachse des Maulteils 19 angeordnet ist. Das Betätigungsglied 31 ist einstückig an dem Maulteil 19, 21 vorgesehen. Dieses Betätigungsglied 31 ist stabförmig ausgebildet und umfasst einen gekrümmten Verlauf. Im Wesentlichen ist das stabförmige Betätigungsglied 31 im Querschnitt gesehen rechteckig oder quadratisch ausgebildet und umfasst eine obere und untere Führungsfläche 41, 42 die koaxial zueinander verlaufen. Am Ende des Betätigungsgliedes ist ein Führungszapfen 43 vorgesehen, der in Richtung der Längsmittelachse des Maulteils 19 hervorsteht. Zwischen dem Führungszapfen 43 und einem Stegabschnitt 44 ist ein Rücksprung 45 vorgesehen, um einen Freiraum zu bilden, damit ein Schlitten 47 (Figur 9 und 10) an dem Führungszapfen 43 angreifen kann. Das zweite Maulteil 21 ist analog zum ersten Maulteil 19 ausgebildet. Lediglich ist die Krallung im Arbeitsbereich 38 ist derart angepasst, dass das erste und zweite Maulteil 19, 21 in die in Figur 3 dargestellte Schließstellung 28 übergeführt werden können.

In Figur 7 ist schematisch vergrößert ein distaler Bereich der ersten beziehungsweise zweiten Körperhälfte 34, 35 des Schaftkörpers 16 dargestellt, wobei dies nachfolgend für die erste Körperhälfte 34 beschrieben wird und analog für die zweite Körperhälfte 35 gilt. An die Stirnfläche 32 angrenzend ist eine erste Kulisse 49 vorgesehen, welche einen gekrümmten Verlauf umfasst. Die Kulisse mündet in die Stirnfläche 32. Von dort aus erstreckt sich die Kulisse bogensegmentförmig in Richtung proximales Ende. Diese Kulisse 49 umfasst eine Laufbahn 51, welche gegenüber der Stirnfläche 32 zurückversetzt ist. Die Laufbahn 51 ist durch Seitenwandabschnitte 52, 53 begrenzt. Dadurch wird eine seitliche Führung des Betätigungsgliedes 31 ermöglicht, so dass dieses im geöffneten Zustand gemäß Figur 4 anordenbar. Die Laufbahn 51 verjüngt sich in proximaler Richtung, jedoch dient auch der verjüngte Bereich der Laufbahn 51 zur Aufnahme und Anlage der Führungsfläche 42 des Maulteils 19.

Am inneren Ende der Laufbahn 63 ist eine Vertiefung 54 (Figur 8 und 9) vorgesehen, welche zur Anordnung und Aufnahme eines Kontaktelementes 56 eines elektrischen Leiters 57 dienen kann, um einen Pol an dem Maulteil 19 anzuschließen. Im Anschluss daran in proximaler Richtung ist eine Schlittenführung 59 angeordnet.

Benachbart zur Kulisse 49 und vorzugsweise durch einen Steg 61 getrennt ist eine zweite Kulisse 60 vorgesehen. Diese zweite Kulisse 60 entspricht der ersten Kulisse 49. Diese ist punktsymmetrisch zur Längsachse des Schaftkörpers 16 ausgebildet. Von der Kulisse 60 ist in der Körperhälfte 34, 35 eine Führungsbahn 63 eingebracht. Diese Führungsbahn 63 ist durch Seitenwände 64, 65 in Analogie zu den Seitenwänden 52, 53 begrenzt. Entlang dieser Führungsbahn 63 ist die Führungsfläche 41 des Betätigungsgliedes 31 vom zweiten Maulteil 21 gekühlt.

Auch diese Laufbahn 63 tritt distalseitig aus der Stirnfläche 32 aus.

Nach dem Zusammensetzen der ersten und zweiten Körperhälfte 34, 35 wird die Kulisse 49 durch die Laufbahn 51, die Führungsbahn 63 gebildet. Analoges gilt für die zweite Kulisse 60. Somit wird das Betätigungsglied 31 zwei einander gegenüberliegende Seitenflächen 52, 53 beziehungsweise 63, 64 als auch durch die einander zugeordnete Laufbahn 51 und Führungsbahn 63 geführt. Jedes Betätigungsglied 31 des Maulteils 19, 21 ist über eine in der geschlossenen Stirnfläche 32 des Schaftkörpers 16 vorgesehenen Öffnung herausgeführt und greift über diese Öffnung in die Kulisse 49, 60 ein. Bevorzugt ist zwischen der Kulisse 49 und 60 der Steg 61 angeordnet, so dass bei einer Kontaktierung der Maulteile 19, 21 mittels eines Pols eine elektrische Kontaktierung zwischen den beiden Betätigungsgliedern 31 oder den Maulteilen 19, 21 verhindert ist.

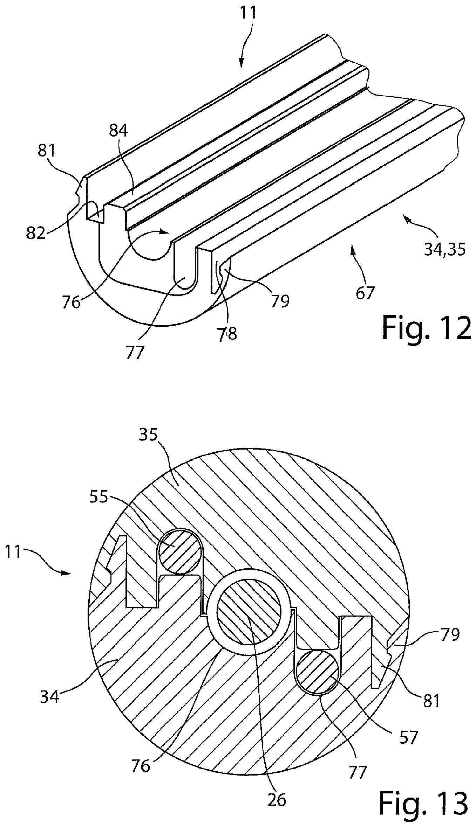

In Figur 8 ist ein gegenüber Figur 7 größerer distaler Bereich der ersten Körperhälfte 34 dargestellt. Aus dieser Darstellung geht die gesamte Länge der Schlittenführung 59 hervor. Proximalseitig schließt sich daraufhin ein Führungsabschnitt 67 an, durch welchen die Zug-/Druckstange 26 sowie die elektrischen Leiter 57 geführt sind. Dies wird nachfolgend noch näher anhand der Figuren 12 und 13 beschrieben.

In Figur 9 ist ein distaler Endbereich des Schaftes 11 dargestellt, wobei die erste Körperhälfte 34 von der zweiten Körperhälfte 35 abgenommen ist, um den Aufbau und die Funktionsweise darzustellen. In der Schlittenführung 59 ist der Schlitten 47 eingesetzt, der mit der Zug-/Druckstange 26 beispielsweise durch einen Stift 68 antriebsverbunden ist. Der Schlitten 47 weist in seinem vorderen zur Stirnfläche 32 weisenden Endbereich zwei einander gegenüberliegende Führungsnuten 71 auf, in welche jeweils der Führungszapfen 43 des Betätigungsgliedes 31 vom ersten und zweiten Maulteil 19, 21 angeordnet und geführt ist. Das erste Maulteil 19 liegt mit der Führungsfläche 42 des Betätigungsgliedes 31 in der Kulisse 49. Das zweite Maulteil 21 liegt mit der Führungsfläche 41 am Betätigungsglied 31 in der zweiten Kulisse 60. Die Betätigungsglieder 31 des ersten und zweiten Maulteils 32 sind vollständig getrennt zueinander von dem vorderen distalen Endbereich der ersten Körperhälfte aufgenommen.

Da der vordere Endbereich der ersten Körperhälfte 34 identisch zur zweiten Körperhälfte 35 ausgebildet ist, wird offensichtlich, dass bei dem geschlossenen Schaftkörper 16 die Laufbahn 51 an der Führungsfläche 42 des zweiten Maulteils und die Führungsbahn 63 an der Führungsfläche 41 des ersten Maulteils 19 anliegt. Dadurch sind zwei geschlossene Kulissen 49, 60 gebildet, die getrennt zueinander angeordnet sind und auch jeweils eine getrennte Bahnführung für die jeweiligen Betätigungsglieder 31 des ersten und zweiten Maulteils 19, 21 bilden. Der Bereich 45 am Betätigungsglied 31 ermöglicht, dass ein stirnseitiges Ende 73 des Schlittens 47 kollisionsfrei verfahren werden kann.

Sobald die Griffteile 24, 25 die Handhabungseinrichtung 23 aufeinander zubewegt werden, wird die Zug-/Druckstange 26 in distaler Richtung verfahren, wodurch der Schlitten 47 auf die Stirnfläche 32 des Schaftkörpers 16 zubewegt wird. Dabei wird eine Schwenkbewegung des ersten und zweiten Maulteils 19, 21 entlang der jeweiligen Kulisse 49, 60 eingeleitet, wobei durch die rechtwinklig zur Verfahrbewegung des Schlittens 47 ausgerichteten Führungsnuten 71 die Schubbewegung in eine Öffnungsbewegung der Maulteile 19, 21 übergeführt wird. Die Betätigungsglieder 31 werden somit entlang der Kulisse 49, 60 ausgefahren, wodurch das erste und zweite Maulteil 19, 21 sich öffnet und dies in Figur 11 dargestellt ist.

In Figur 11 ist eine schematische Schnittansicht des distalen Bereichs vom Schaft 11 und des Werkzeuges 18 dargestellt. Entlang des Schaftes 11 im Schaftkörper 16 ist für jeden Pol die elektrische Leitung 57 geführt, welche mittels des Kontaktelements 56 eine Führungsfläche 41 des zweiten Maulteils 21 kontaktiert. Dieses Kontaktelement 56 ist bevorzugt als Schleifkontakt ausgebildet. Zudem ist das Kontaktelement 56 federartig gekrümmt ausgebildet, so dass dies mit Sicherheit aufgrund der Federkraft an der Führungsfläche 41 anliegt. Eine analoge Anordnung ist für den zweiten Pol vorgesehen, in dem ein solches Kontaktelement 56 an der Führungsfläche 41 des ersten Maulteils 19 anliegt. Somit ist jedes Maulteil 19, 21 mit einem Pol bestromt. Unabhängig, ob die Öffnungs- oder Schließstellung 28, 29 der Maulteile 19, 21 eingenommen ist, bleibt die Kontaktierung gegeben. Durch die getrennte Anordnung der Betätigungsglieder 31 in jeweils einer Kulisse 49, 60 sind keine zusätzlichen elektrisch isolierenden Maßnahmen vorzusehen. Die erste und zweite Körperhälfte 34, 35 sind bevorzugt aus einem nicht leitenden Material ausgebildet. Sofern für die ersten und zweiten Körperhälften 34, 35 ein leitendes Material zum Einsatz kommen sollte, kann die Kulisse 49, 60 mit einer elektrisch nicht leitenden Beschichtung ausgekleidet werden.

Gemäß einer weiteren Ausführungsform kann das Betätigungsglied 31 bei einer geschlossenen Anordnung der Maulteile 19, 21 nur in dem Bereich der Führungsfläche 41 leitend ausgebildet sein, in dem gemäß Figur 11 das Kontaktelement 56 bei einer geschlossenen Anordnung der Maulteile anliegt. Sobald eine Öffnungsbewegung der Maulteile 19, 21 aus der Schließstellung 29 der Maulteile 19, 21 durch den Schlitten 47 angesteuert wird, kann eine sich von dem Kontaktelement 56 bis zum Ende des Betätigungsgliedes 31 sich erstreckende Führungsfläche nicht leitend ausgebildet sein, insbesondere durch eine nicht leitende Beschichtung oder ein nicht leitendes Material. Dadurch wird der Stromfluss unterbrochen und an den Maulteilen 19, 21 liegt in einer Öffnungsstellung kein Strom an. Dies ermöglicht beispielsweise, dass die Maulteile 19, 21 ein Gewebe während dem Überführen in eine Schließstellung erfassen können, jedoch erst in der Schließstellung die Maulteile 19, 21 zum Koagulieren bestromt werden.

Bei den vorstehenden Ausführungsformen sind die Kulissen 49 der beiden Betätigungsglieder 31 der Maulteile 19 und 21 punktsymmetrisch zu einer Längs- Mittelachse ausgebildet, so dass eine synchrone Öffnungs- und Schließbewegung der Maulteile 19, 21 möglich ist.

Alternativ kann vorgesehen sein, dass die beiden beispielsweise dargestellten Kulissen 49 und 60 in der Länge, der Krümmung und/oder im Verlauf voneinander abweichend ausgebildet sind. Dadurch kann eine asynchrone Schließbewegung der Maulteile 19, 21 angesteuert sein. Beispielsweise kann eine Kulisse 49 eine flachere Krümmung als die andere Kulisse aufweisen. Des Weiteren kann die Länge der Führungsbahnen der Kulissen 49, 60 unterschiedlich ausgebildet sein, so dass die Öffnungsweite der Maulteile 19, 21 voneinander abweichend ist.

In Figur 12 ist eine perspektivische Ansicht des Führungsabschnitts 67 im mittleren Bereich des Schaftes 11 dargestellt. Durch diesen Aufbau wird ermöglicht, dass die erste und zweite Körperhälfte 34, 35 identisch ausgebildet sein können und dennoch wie dies in Figur 13 dargestellt ist jeweils eine getrennte Anordnung und Aufnahme der elektrischen Leiter 57 und der Zug-/Druckstange 26 ermöglichen. Die erste beziehungsweise zweite Körperhälfte 34, 35 umfasst eine zentrale Aufnahme 76, welche bevorzugt als Halbkreis ausgebildet ist. In einer radialen Richtung nach außen ist eine Vertiefung 77 vorgesehen, welche U-förmig ausgebildet ist, um den elektrischen Leiter aufzunehmen. Benachbart ist ein Kanal 78 ausgebildet, der an einer Innenwand eine Kontur für ein positives Rastelement 79 bildet. Die freigeschnittene Kontur ist die eines negativen Rastelementes 81, welches auf der gegenüberliegenden Seite körperlich ausgebildet ist. Zwischen der Aufnahme 76 und einer weiteren Vertiefung 82 ist eine Erhöhung 84 vorgesehen. Sobald beide Körperhälften 34, 35 einander zugeführt und miteinander verklipst sind, ergibt sich eine Querschnittsdarstellung gemäß Figur 13. Daraus ist ersichtlich, dass zum einen eine kreisrunde geschlossene Aufnahme 76 für die Zug-/Druckstange 26 gebildet wird, sowie geschlossene Aufnahmebereiche für die elektrischen Leitungen 57.

Die in Figur 5 dargestellte Körperhälfte 34, 35 ist für den Einsatz des Schaftes 11 bei einem mono- oder bipolaren medizinischen Instrument 12 vorgesehen. Sofern weder die Elektrode 55 noch die Elektrode 57 eingelegt werden, kann dieser Schaft auch für ein nicht bestromtes medizinisches Instrument, beispielsweise eine Fasszange, eingesetzt werden. In einem solchen Anwendungsfall können die Erhöhung 84 und Vertiefung 77 auch entfallen, so dass der schematische Querschnitt der Körperhälfte 34, 35 vereinfacht ist.

Ebenso kann ein universeller einsetzbarer Schaft 11 ausgebildet sein, der den Querschnitt gemäß Figur 12, 13 aufweist und auch darin neben der Zug-/Druckstange 26 Elektroden 55, 57 aufnimmt, wobei dieser Schaft 11, sofern keine Bestromung gewünscht ist, auch für eine Handhabungseinrichtung 23 verwendbar ist, welche ohne elektrischen Anschluss ausgebildet ist. Der Schaft 11 ist auch für herkömmliche chirurgische Instrumente, insbesondere Fasszangen, einsetzbar.

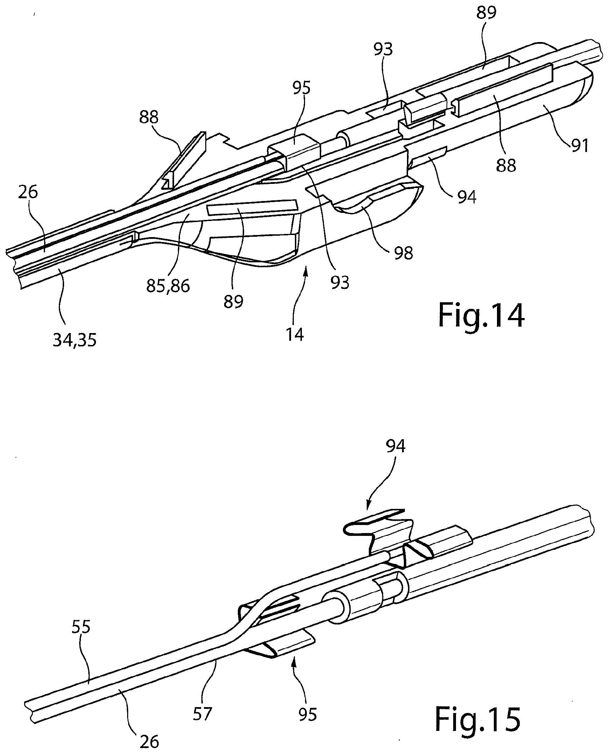

In Figur 14 ist eine schematische Ansicht auf einen proximalen Endbereich der Körperhälfte 34, 35 des Schaftes 11 dargestellt. Daraus wird ersichtlich, dass auch das Anschlussstück 14 einteilig mit dem Schaftbereich der Körperhälfte 34, 35 verbunden ist. Die Hälften 85, 86 des Anschlussstückes 14, die vorzugsweise einteilig mit dem Schaftbereich der Körperhälften 34, 35 ausgebildet sind, weisen ebenfalls Rastelemente 88, 89 auf. Diese Rastelemente 88, 89 liegen einander gegenüber und sind komplementär ausgebildet, so dass beim Zusammenfügen der beiden Hälften 85, 86 die beiden Rastelemente 88, 89 ineinandergreifen und ein geschlossenes Anschlussstück 14 bilden.

Am proximalen Ende des Schafts 12 beziehungsweise der Körperhälfte 34, 35 ist jeweils ein Führungsabschnitt 91 angebracht, der zur drehbaren Anordnung innerhalb der Handhabungseinrichtung 23 vorgesehen ist.

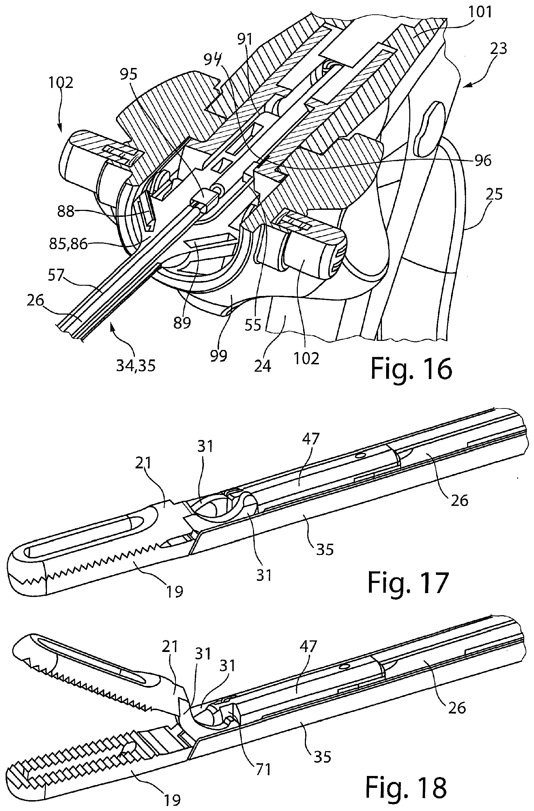

Des Weiteren ist in der Hälfte 85, 86 des Anschlussstücks 16 als auch in jedem Führungsabschnitt 91 der Körperhälfte 34, 35 eine Aussparung 93 vorgesehen. In diese Aussparung 93 können Kontaktelemente 94, 95 eingesetzt werden, welche elektrisch leitend mit den Elektroden 55, 57 verbunden sind. Das Kontaktelement 94, welches im Bereich des Führungsabschnitts 91 vorgesehen ist, liegt bevorzugt an einer äußeren Umfangsfläche des Führungsabschnitts 91, so dass nach dem Einsetzen des Führungsabschnitts 91 in die Handhabungseinrichtung 23 dadurch eine elektrische Kontaktierung 96, beispielsweise durch einen leitenden Ring im Gehäuse 101 der Handhabungseinrichtung 23 (Figur 16), erfolgt. Das Kontaktelement 95 kann eine elektrische Kontaktierung zwischen der Zug-/Druckstange 26 und der Elektrode 57 ermöglichen.

Die Hälfte 85, 85 des Anschlussstücks weist des Weiteren Rastflächen 98 auf, welche nach dem Zusammensetzen der Hälften 85, 86 eine Art Öse bilden, so dass darauffolgend ein lösbares Rastelement 102, insbesondere ein Rastpin oder ein Clips einer Drehmuffe 99, an der Handhabungseinrichtung 23 daran angreifen kann.

In Figur 15 ist perspektivisch und vergrößert die Zug-/Druckstange 26 sowie dieser zugeordnet das Kontaktelement 94 mit der elektrisch verbundenen Elektrode 55 als auch die Positionierung des weiteren Kontaktelementes 95, welches mit der Elektrode 57 in Verbindung steht, dargestellt.

In Figur 16 ist eine perspektivische Schnittansicht einer Handhabungseinrichtung 23 und ein proximaler Bereich des Schaftes 11 dargestellt, der in die Handhabungseinrichtung 23 eingesetzt ist. An der Drehmuffe 99, welche frei drehbar zum Gehäuse 101 der Handhabungseinrichtung drehbar angeordnet ist, sind lösbare Rastelemente 102 vorzugsweise einander gegenüberliegend vorgesehen. Hierbei kann es sich um federgelagerte Rastpins handeln, welche in die Rastaufnahme 98, die als eine geöffnete Schlüssellochanordnung ausgebildet sein kann, eingreifen. Dadurch kann das proximale Ende des Schaftes 11 lösbar, jedoch in axialer Richtung lagegesichert aufgenommen werden. Der Führungsabschnitt 91 ist drehbar in dem Gehäuse 101 gelagert. In einer Umfangsfläche im Gehäuse 101 ist gleichzeitig ein elektrischer Kontaktring 96 vorgesehen, so dass das Kontaktelement 34 unabhängig der rotatorischen Ausrichtung elektrisch leitend mit dem Anschluss 30 verbunden ist. Das proximale Ende der Zug-/Druckstange 26 wird in bekannter Weise mit einem bewegbaren Griffteil 24 der Handhabungseinrichtung 23 durch eine lösbare Rast-, Klemm-, Steckverbindung aufgenommen.

Bei dieser beispielhaften Ausführungsform der Handhabungseinrichtung 31 kann die Stromzuführung für eine der beiden Elektroden über den Führungsabschnitt 91 am Schaft 11 mit dem Gehäuse 101 erfolgen. Die Bestromung für die weitere Elektrode kann beispielsweise über die Zug-/Druckstange 26 erfolgen, indem diese am proximalen Ende im Handgriff elektrisch kontaktiert ist. Die Kontaktelemente 94, 95 sind im Anschlussstück 14 getrennt zueinander angeordnet, so dass eine Durchkontaktierung verhindert ist. Auch die Positionierung der Elektroden 55, 57 sind getrennt zueinander ausgehend von den jeweiligen Kontaktelementen 94, 95 der Körperhälften 34, 35 im Schaft 11 geführt.

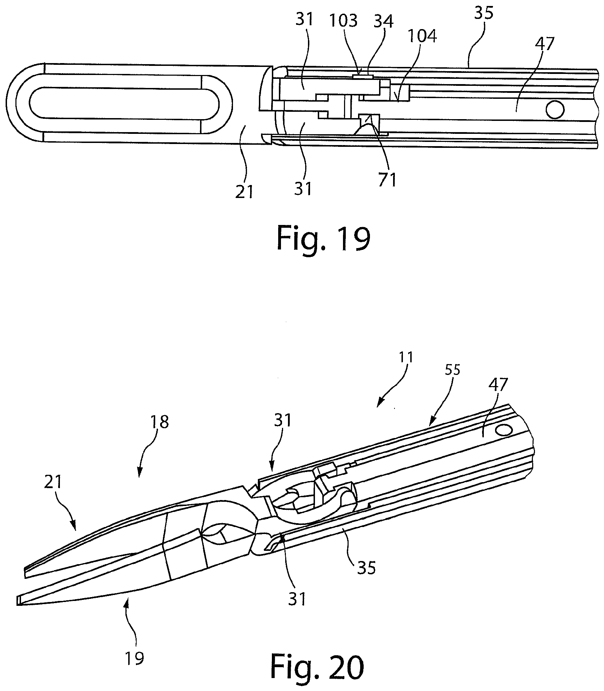

In Figur 17 ist eine perspektivische Ansicht einer alternativen Ausführungsform des Werkzeugs 18 am Schaft 11 dargestellt, wobei eine obere Körperhälfte 34 von der unteren Körperhälfte 35 abgenommen ist. Bei dieser Ausführungsform ist vorgesehen, dass das erste Maulteil 19 als feststehendes Maulteil ausgebildet ist und das zweite Maulteil 21 als bewegliches Maulteil, wie dies in Figur 18 dargestellt ist. Die Figur 19 zeigt eine Draufsicht auf die Darstellung des Schaftes 11 in Figur 17.

Im Hinblick auf die Ausgestaltung des Maulteils 21 sowie des Schlittens 47 für das bewegte zweite Maulteil 21 wird auf die vorstehende Beschreibung Bezug genommen.

Das erste feststehende Maulteil 19 weist lediglich dahingehend von dem bewegten Maulteil 21 ab, dass der Führungszapfen 43 auf einer gegenüberliegenden Seite des Betätigungsgliedes 31 angeordnet ist, das heißt, dass der Führungszapfen 43 nicht zum Schlitten 47 weist, sondern in entgegengesetzte Richtung. Der Führungszapfen 43 ist durch eine U-förmige Ausnehmung 103 in der Körperhälfte 35 aufgenommen, welche zur Trennebene zwischen den beiden Körperhälften 34, 35 hin offen ausgebildet ist. Im Übrigen liegt das Betätigungsglied 19 mit seiner Führungsfläche 41, 42 an den Flächen 51 beziehungsweise 63 der ersten und zweiten Körperhälfte 34, 35 an. Durch das Einsetzen des Führungszapfens 43 in die U-förmige Ausnehmung 103 an der Körperhälfte 35 wird eine Schwenkbewegung gesperrt. Der Schlitten 47 weist anstelle der Führungsnut 71, wie dies in Figur 10 dargestellt ist, eine vergrößerte Ausnehmung 104 auf, so dass der Schlitten 47 längs des Schafts 11 verfahrbar ist, um das bewegte zweite Maulteil 21 zu betätigen, jedoch getrennt zum Betätigungsglied 31 des feststehenden ersten Maulteils 19 ausgebildet ist.

In Figur 20 ist eine alternative Ausführungsform eines Werkzeuges 18 für einen Schaft 11 dargestellt. Beispielsweise ist das erste und zweite Maulteil 19, 21 schneidend ausgebildet, so dass eine Scherenfunktion gegeben ist. Weitere alternative Ausgestaltungen der Maulteile 19, 21 können ebenso vorgesehen sein. Diese können auch wahlweise an dem Schaft 11 eingesetzt werden. Es ist lediglich erforderlich, dass die Betätigungsglieder 31 auf die Kulissen 49, 60 angepasst sind.

## Patentansprüche

1. Schaft für ein medizinisches Instrument (12), insbesondere ein mono- oder bipolares medizinisches Instrument, mit einem Schaftkörper (16), der am distalen Ende ein Werkzeug (18) aufnimmt, welches ein erstes Maulteil (19) und ein zweites Maulteil (21) aufweist, die mit einer im Schaftkörper (16) geführten Zug-/Druckstange (26) relativ zueinander von einer Öffnungsstellung (29) in eine Schließstellung (28) überführbar sind und der am proximalen Ende ein Anschlussstück (14) zum Befestigen an einer Handhabungseinrichtung (23) aufweist, durch welche die Zug-/Druckstange (26) hin und her bewegbar ist, und das zumindest eine Maulteil (19, 21) am distalen Ende im Schaftkörper (16) durch zumindest eine Kulisse (49, 60) schwenkbar geführt ist, welche eine bogensegmentförmige Laufbahn (51) und eine koaxial dazu verlaufende Führungsbahn (63) aufweist und ein Betätigungsglied (31) des Maulteils (19, 21) komplementär gekrümmt ausgebildet und mit einer äußeren und inneren Führungsfläche (41, 42) entlang der Laufbahn und der Führungsbahn (51, 63) in der Kulisse (49, 60) geführt ist, **dadurch gekennzeichnet, dass** im Schaftkörper (16) ein Schlitten (47) entlang der Längsachse des Schaftkörpers (16) verfahrbar geführt ist, der durch die Zug-/Druckstange (26) bewegbar ist und das zumindest eine Maulteil (16, 21) in eine Öffnungsstellung (29) und eine Schließstellung (28) überführt.

2. Schaft nach Anspruch 1, **dadurch gekennzeichnet, dass** jedes Maulteil (19, 21) am distalen Ende im Schaftkörper (16) durch jeweils eine Kulisse (49, 60) schwenkbar geführt ist, welche vorzugsweise innerhalb eines Außenumfangs des Schaftkörpers (16) angeordnet sind.

3. Schaft nach einem der vorhergehenden Ansprüche, **dadurch gekennzeichnet, dass** zwei zueinander benachbart und getrennt zueinander angeordnete Kulissen (49, 60) am distalen Ende des Schaftkörpers (18) vorgesehen sind, in welcher jeweils ein Betätigungsglied (31) des Maulteils (19, 21) angeordnet ist und vorzugsweise die zumindest eine Kulisse (49, 60) sich ausgehend von einer Stirnfläche (32) des Schaftkörpers (16) in dem Schaftkörper (16) erstreckt, und/oder die beiden Kulissen (49, 60) punktsymmetrisch zur Längsmittelachse des Schaftkörpers (18) ausgerichtet sind.

4. Schaft nach einem der vorhergehenden Ansprüche, **dadurch gekennzeichnet, dass** die Betätigungsglieder (31) der zwei das Werkzeug (18) bildenden Maulteile (19, 21) gegenläufig gekrümmt sind.

5. Schaft nach einem der vorhergehenden Ansprüche, **dadurch gekennzeichnet, dass** die Kulisse (49, 60) geschlossen ausgebildet ist und vorzugsweise einen rechteckförmigen, quadratischen, runden, ovalen oder halbkreisförmigen Querschnitt aufweist.

6. Schaft nach einen der vorherigen Ansprüche, **dadurch gekennzeichnet, dass** das Betätigungsglied (31) des Maulteils (19, 21) stabförmig gekrümmt ausgebildet ist und vorzugsweise einen an die Kulisse (49, 69) angepassten Querschnitt aufweist.

7. Schaft nach einem der vorhergehenden Ansprüche, **dadurch gekennzeichnet, dass** das Betätigungsglied (31) des Maulteils (19, 21) außermittig zur Längsmittelachse des Arbeitsbereiches des Maulteils (19, 21) oder seitlich versetzt zum Arbeitsbereich (38) des Maulteils (19, 21) vorgesehen ist.

8. Schaft nach einem der vorhergehenden Ansprüche, **dadurch gekennzeichnet, dass** am freien Ende des Betätigungsgliedes (31) des Maulteils (19, 21) ein Führungszapfen (43) vorgesehen ist, der in einer Führungsnut (71) am Schlitten (47) eingreift und vorzugsweise der Schlitten (47) zwei einander gegenüberliegende, nach außen offene Führungsnuten (71) aufweist und der Schlitten (47) im Bereich der Führungsnuten (71) zwischen den beiden Kulissen (49, 60) verfahrbar ist.

9. Schaft nach Anspruch 8, **dadurch gekennzeichnet, dass** die Führungsnuten (71) des Schlittens (47) rechtwinklig zur Antriebsbewegung des Schlittens (47) entlang der Längsachse des Schaftkörpers (16) ausgerichtet sind.

10. Schaft nach einem der Ansprüche 8 bis 9, **dadurch gekennzeichnet, dass** der Schlitten (47) einen länglichen Führungskörper aufweist, der verdrehsicher in einer Schlittenführung (59) des Schaftkörpers (16) verfahrbar ist.

11. Schaft nach einem der vorhergehenden Ansprüche, **dadurch gekennzeichnet, dass** die Maulstücke (19, 21) aus Metall, Kunststoff oder Keramik oder aus einem nichtleitenden Material mit einer zumindest bereichsweise leitenden Beschichtung oder leitenden Komponente hergestellt sind.

12. Schaft nach einem der vorhergehenden Ansprüche, **dadurch gekennzeichnet, dass** zumindest eine Außenfläche des Betätigungsgliedes (31) vorzugsweise an einer Führungsfläche (41, 42) des Betätigungsgliedes (31) der Maulteile (19, 21) eine Kontaktstelle für eine im Schaftkörper (16) geführte Elektrode (55) gebildet ist und vorzugsweise die im Schaftkörper (16) vorgesehene Elektrode (55) getrennt zum verfahrbaren Schlitten (47) angeordnet ist.

13. Schaft nach Anspruch 12, **dadurch gekennzeichnet, dass** in dem Schaftkörper (18) zumindest eine, vorzugsweise zwei Elektroden (55) geführt sind, welche jeweils einen elektrischen Leiter (57) mit einem Kontaktelement (56) umfassen und das Kontaktelement (56) mit dem Betätigungsglied (31) des Maulteils (19, 21) kontaktiert ist.

14. Schaft nach einem der vorhergehenden Ansprüche, **dadurch gekennzeichnet, dass** die Maulteile (19, 21) einen schneidenden, greifenden, klemmenden oder löffelmaulartigen Arbeitsbereich (28) aufweisen oder als Pinzette oder Nadelelektrode ausgebildet sind.

15. Chirurgisches Instrument, insbesondere mono- oder bipolares medizinisches Instrument mit einer Handhabungseinrichtung (23), **dadurch gekennzeichnet, dass** ein Schaft (11) nach einem der Ansprüche 1 bis 14 an der Handhabungseinrichtung (23) anschließbar ist.

## Claims

1. Shaft for a medical instrument (12), in particular a mono- or bipolar medical instrument, having a shaft body (16) which receives a tool (18) at the distal end, said tool having a first mouth part (19) and a second mouth part (21) which are transferrable relative to each other from an opening position (29) into a closing position (28) with a pull/push rod (26) which is guided in the shaft body (16), the shaft body having a connecting piece (14) on the proximal end for fastening to a handling device (23), by means of which the pull/push rod (26) is moveable backwards and forwards, and the at least one mouth part (19, 21) at the distal end in the shaft body (16) is pivotably guided by at least one connecting link (49, 60) which has an arcuate segment-shaped track (51) and a guideway (63) running coaxially thereto, and an actuating member (31) of the mouth part (19, 21) is formed to be curved in a complementary manner and is guided, with an outer and inner guide surface (41, 42), along the track and the guideway (51, 63) in the connecting link (49, 60), **characterised in that**, in the shaft body (16), a carriage (47) is displaceably guided along the longitudinal axis of the shaft body (16), said carriage being moveable by means of a pull/push rod (26) and transferring at least one mouth part (16, 21) into an opening position (29) and a closing position (28).

2. Shaft according to claim 1, **characterised in that** each mouth part (19, 21) is guided in each case in the in the shaft body (16) at the distal end by a connecting link (49, 60), said connecting links being preferably arranged within an outer periphery of the shaft body (16).

3. Shaft according to one of the preceding claims, **characterised in that** two connecting links (49, 60) which are arranged adjacent to each other and separately from each other are provided at the distal end of the shaft body (18) an actuating member (31) of the mouth part (19, 21) being arranged in said connecting links, and preferably the at least one connecting link (49, 60) extends in the shaft body (16) starting from an end face (32) of the shaft body (16), and/or the two connecting links (49, 60) are oriented pointsymmetrically to the longitudinal central axis of the shaft body (18).

4. Shaft according to one of the preceding claims, **characterised in that** the actuating member (31) is curved in the opposite direction to the two mouth parts (19, 21) forming the tool (18).

5. Shaft according to one of the preceding claims, **characterised in that** the connecting link (49, 60) is formed to be closed and preferably has a rectangular, square, round, oval or semi-circular cross-section.

6. Shaft according to one of the preceding claims, **characterised in that** the actuating member (31) of the mouth part (19, 21) is formed to be curved in a rod-like manner and preferably has a cross-section which is adapted to the connecting link (49, 69).

7. Shaft according to one of the preceding claims, **characterised in that** the actuating member (31) of the mouth part (19, 21) is provided eccentrically relative to the longitudinal central axis of the mouth part (19, 21) or offset laterally relative to the working region (38) of the mouth part (19, 21).

8. Shaft according to one of the preceding claims, **characterised in that** a guide pin (43) is provided on the free end of the actuating member (31) of the mouth part (19, 21), said guide pin engaging in a guide groove (71) on the carriage (47), and preferably the carriage (47) has two opposite guide grooves (71) which are open outwardly and the carriage (47) is displaceable in the region of the guide grooves (71) between the two connecting links (49, 60).

9. Shaft according to claim 8, **characterised in that** the guide grooves (71) of the carriage (47) are oriented at a right angle to the drive movement of the carriage (47) along the longitudinal axis of the shaft body (16).

10. Shaft according to one of claims 8 to 9, **characterised in that** the carriage (47) has an elongated guide body which is non-rotatably displaceable in a carriage guide (59) of the shaft body (16).

11. Shaft according to one of the preceding claims, **characterised in that** the mouth pieces (19, 21) are produced from metal, plastic or ceramic or from a non-conductive material having an at least regionally conductive coating or conductive components.

12. Shaft according to one of the preceding claims, **characterised in that** at least one outer surface of the actuating member (31) is formed a contact point for an electrode (55) guided in the shaft body (16), preferably on a guiding surface (41, 42) of the actuating member (31) of the mouth parts (19, 21), and preferably the electrode (55) provided in the shaft body (16) is arranged separately to the displaceable carriage (47).

13. Shaft according to claim 12, **characterised in that** at least one, preferably two electrodes (55) are guided in the shaft body (18), which respectively comprise an electrical conductor (57) having a contact element (56) and the contact element (56) is contacted with the actuating member (31) of the mouth part (19, 21).

14. Shaft according to one of the preceding claims, **characterised in that** the mouth parts (19, 21) have a cutting, gripping, clamping or spoon mouth-shaped working region (28) or are formed as forceps or as a needle electrode.

15. Surgical instrument, in particular mono- or bipolar medical instrument, having a handling device (23), **characterised in that** a shaft (11) according to one of claims 1 to 14 is connecteable to the handling device (23).

## Revendications

1. Tige pour un instrument médical (12), en particulier pour un instrument médical monopolaire ou bipolaire, pourvue d'un corps de tige (16) lequel reçoit, à son extrémité distale, un outil (18) qui présente un premier élément de mâchoire (19) et un deuxième élément de mâchoire (21) lesquels peuvent être déplacés l'un par rapport à l'autre entre une position d'ouverture (29) et une position de fermeture (28) grâce à une barre de traction/poussée (26) guidée dans le corps de tige (16), et lequel présente, à son extrémité proximale, une pièce de raccordement (14) destinée à être fixée à un dispositif de manipulation (23) grâce auquel la barre de traction/poussée (26) peut être déplacée en avant et en arrière et ledit au moins un élément de mâchoire (19, 21) prévu à l'extrémité distale est guidé dans le corps de tige (16) par au moins une coulisse (49, 60) de manière à pouvoir pivoter, laquelle présente un chemin de course (51) en forme de segment d'arc et un chemin de guidage (63) qui s'étend de manière coaxiale à celui-ci, et un mécanisme d'actionnement (31) de l'élément de mâchoire (19, 21) qui est réalisé de manière à présenter une forme incurvée complémentaire et est guidé dans la coulisse (49, 60) le long du chemin de course et du chemin de guidage (51, 63) grâce à une surface de guidage respectivement extérieure et intérieure (41, 42), **caractérisée en ce que** dans corps de tige (16), un chariot (47) qui peut être déplacé par la barre de traction/poussé (26) est guidé de manière déplaçable le long de l'axe longitudinal dudit corps de tige (16), ce qui fait passer ledit au moins un élément de mâchoire (16, 21) dans une position d'ouverture (29) et une position de fermeture (28).

2. Tige selon la revendication 1, **caractérisée en ce que** chaque élément de mâchoire (19, 21) est guidé de manière pivotante à l'extrémité distale du corps de tige (16) respectivement grâce à une coulisse (49, 60) disposée de préférence à l'intérieur d'une circonférence extérieure dudit corps de tige (16).

3. Tige selon l'une quelconque des revendications précédentes, **caractérisée en ce qu'**à l'extrémité distale du corps de tige (18) sont prévues deux coulisses (49, 60) contiguës et séparées l'une de l'autre dans chacune desquelles est disposé un mécanisme d'actionnement (31) de l'élément de mâchoire (19, 21), et **en ce que** de préférence ladite au moins une coulisse (49, 60) s'étend dans le corps de tige (16) à partir d'une face frontale (32) dudit corps de tige (16), et/ou que les deux coulisses (49, 60) sont orientées en symétrique ponctuelle par rapport à l'axe central longitudinal du corps de tige (18).

4. Tige selon l'une quelconque des revendications précédentes, **caractérisée en ce que** les mécanismes d'actionnement (31) des deux éléments de mâchoire (19, 21) formant l'outil (18) sont incurvés en sens inverse.

5. Tige selon l'une quelconque des revendications précédentes, **caractérisée en ce que** la coulisse (49, 60) est réalisée de manière fermée et présente de préférence une section transversale rectangulaire, carrée, ronde, ovale ou semi-circulaire.

6. Tige selon l'une quelconque des revendications précédentes, **caractérisée en ce que** le mécanisme d'actionnement (31) de l'élément de mâchoire (19, 21) est réalisé en forme de bâtonnet incurvé et présente de préférence une section transversale adaptée à la coulisse (49, 69).

7. Tige selon l'une quelconque des revendications précédentes, **caractérisée en ce que** le mécanisme d'actionnement (31) de l'élément de mâchoire (19, 31) est prévu de manière excentrée par rapport à l'axe central longitudinal de la zone de travail de l'élément de mâchoire (19, 21) ou est prévu de manière à être décalé latéralement par rapport à la zone de travail (38) de l'élément de mâchoire (19, 21) .

8. Tige selon l'une quelconque des revendications précédentes, **caractérisée en ce qu'**à l'extrémité libre du mécanisme d'actionnement (31) de l'élément de mâchoire (19, 21) est prévu un tenon de guidage (43) qui engrène dans une rainure de guidage (71) ménagée dans le chariot (47), et **en ce que** le chariot (47) présente de préférence deux rainures de guidage (71) situées à l'opposé l'une de l'autre et ouvertes vers l'extérieur, et que ledit chariot (47) peut être déplacé dans la zone des rainures de guidage (71), entre les deux coulisses (49, 60).

9. Tige selon la revendication 8, **caractérisée en ce que** les rainures de guidage (71) du chariot (47) sont orientées à angle droit par rapport au mouvement d'actionnement du chariot (47) le long de l'axe longitudinal du corps de tige (16).

10. Tige selon l'une quelconque des revendications 8 à 9, **caractérisée en ce que** le chariot (47) présente un corps de guidage allongé qui peut être déplacé dans un guide de chariot (59) du corps de tige (16) de manière à ne pas tourner.

11. Tige selon l'une quelconque des revendications précédentes, **caractérisée en ce que** les éléments de mâchoire (19, 21) sont réalisés en métal, en matière plastique ou en céramique ou bien en un matériau non conducteur pourvu, au moins dans certaines zones, d'un revêtement conducteur ou d'un composant conducteur.

12. Tige selon l'une quelconque des revendications précédentes, **caractérisée en ce que** sur au moins une surface extérieure du mécanisme d'actionnement (31), de préférence sur une surface de guidage (41, 42) du mécanisme d'actionnement (31) des éléments de mâchoire (19, 21), est formé un point de contact pour une électrode (55) placée dans le corps de tige (16), et **en ce que** de préférence l'électrode (55) prévue dans le corps de tige (16) est disposée de manière séparée du chariot déplaçable (47).

13. Tige selon la revendication 12, **caractérisée en ce que** dans le corps de tige (18) sont placées au moins une, de préférence deux électrodes (55) qui comprennent respectivement un conducteur électrique (57) pourvu d'une pièce de contact (56), et **en ce que** la pièce de contact (56) est mise en contact avec le mécanisme d'actionnement (31) de l'élément de mâchoire (19, 21).

14. Tige selon l'une quelconque des revendications précédentes, **caractérisée en ce que** les éléments de mâchoire (19, 21) présentent une zone de travail (28) coupante, préhensile, de serrage ou de type cuillère ou réalisée sous forme de pincette ou d'électrode aiguille.

15. Instrument chirurgical, en particulier instrument médical monopolaire ou bipolaire, pourvu d'un dispositif de manipulation (23), **caractérisé en ce qu'**une tige (11) selon l'une quelconque des revendications 1 à 14 peut être raccordée au dispositif de manipulation (23).
